# EUROPEAN PATENT APPLICATION

(11) **EP 4 691 367 A2**
(43) Date of publication of application: **11.02.2026**
(21) Application number: 25225913.0
(22) Date of filing: 22.05.2020
(51) Int. Cl.: A61B 5/22

(54) **FORCE PROFILE SENSING AND ASSESSMENT**

(30) Priority: 24.05.2019 US 201962852726 P
(62) Divisional of application: 20814421.2
(71) Applicant: The Regents of The University of Michigan, Ann Arbor, MI 48109 (US)
(72) Inventor: The designation of the inventor has not yet been filed
(74) Representative: Abraham, Richard

(57) **Abstract**

A force assessment device includes a support structure having a periphery, a cover positioned around the support structure and configured to define a surface, and a plurality of sensor elements disposed along the periphery of the support structure, each respective sensor element of the plurality of sensor elements being configured to generate an output signal indicative of force applied to the surface at the respective sensor element. The plurality of sensor elements are distributed across the surface such that the output signals from the plurality of sensor elements are collectively indicative of a force profile along the surface, the force profile providing force position data and force magnitude data correlated with the force position data.

## Description

### CROSS-REFERENCE TO RELATED APPLICATION

This application claims the benefit of U.S. provisional application entitled "Grip Profile Sensing and Assessment," filed May 24, 2019, and assigned Serial No. 62/852,726, the entire disclosure of which is hereby expressly incorporated by reference.

### BACKGROUND OF THE DISCLOSURE

### Field of the Disclosure

The disclosure relates generally to measurement of grip and other surface forces.

### Brief Description of Related Technology

The elbow has collateral ligaments located on the inner and outer sides of the elbow. The ligament on the inside of the elbow is the medial ulnar collateral ligament. The medial ulnar collateral ligament runs from the medial inner side of the upper arm bone (i.e., the humerus) to the medial side of the larger of the two bones in the forearm (i.e., the ulna). The medial ulnar collateral ligament is the primary stabilizer of the elbow during valgus stress motions, such as throwing. Injuries of the medial ulnar collateral ligament range from minor damage and inflammation to a complete tear of the ligament.

The medial ulnar collateral ligament can be ruptured by sudden traumatic accidents, however, more commonly, the medial ulnar collateral ligament undergoes attenuation over time, which can eventually lead to rupture. Such attenuation results from repeated stresses related to the specific repetitive motion of pitching and other throwing sports. For this reason, attenuation and subsequent injury is common among baseball pitchers, javelin throwers, and other throwing athletes, as well as gymnasts who also engage in repetitive valgus motion and stresses across the elbow.

Tears and other injuries to the medial ulnar collateral ligament remain common in baseball pitchers of all ages, from adolescence to the professional. Knowledge of the dynamics of the elbow has yet to provide a comprehensive understanding of how to prevent such injuries. For instance, it is known that the dynamic muscular contribution of the flexor wad is related to the protection of the medial ulnar collateral ligament. However, questions remain regarding the conditions of the flexor wad under which the medial ulnar collateral ligament is injured. Further information regarding the extent to which fatigue of the flexor and extensor wad occurs with pitching, throwing, and swinging sports, is paramount to further understanding of these common pathologies.

### SUMMARY OF THE DISCLOSURE

In accordance with one aspect of the disclosure, a grip assessment device includes a support structure having a periphery, a cover positioned around the support structure and configured to define a grip surface, and a plurality of sensor elements disposed along the periphery of the support structure. Each respective sensor element of the plurality of sensor elements is configured to generate an output signal indicative of force applied to the grip surface at the respective sensor element. The plurality of sensor elements are distributed across the grip surface such that the output signals from the plurality of sensor elements are collectively indicative of a grip profile along the grip surface, the grip profile providing grip position data and grip magnitude data correlated with the grip position data.

In accordance with another aspect of the disclosure, an instrumented baseball includes a core having a periphery, a leather cover wrapped around the core and configured to define a grip surface, a plurality of sensor elements disposed along the periphery of the core, each respective sensor element of the plurality of sensor elements being configured to generate an output signal indicative of force applied to the grip surface at the respective sensor element, and a circuit disposed inside the core, communicatively connected to the plurality of sensor elements, and configured to generate grip profile data based on the output signals.

In accordance with yet another aspect of the disclosure, a method of assessing a baseball grip profile includes obtaining, with a processor, sensor data captured during a pitch by an instrumented baseball, the sensor data being indicative of grip position data and grip magnitude data correlated with the grip position data, generating, with the processor, grip profile data based on the sensor data, implementing, with the processor, a comparison of the grip profile data with preset grip profile data, and providing, with the processor, an assessment of the baseball grip profile based on the comparison.

In accordance with still another aspect of the disclosure, a grip assessment system includes a grip assessment device including a support structure having a periphery, a cover positioned around the support structure and configured to define a grip surface, a plurality of sensor elements disposed along the periphery of the support structure, each respective sensor element of the plurality of sensor elements being configured to generate an output signal indicative of force applied to the grip surface at the respective sensor element, and a control circuit communicatively coupled to the plurality of sensor elements and configured to generate grip profile data based on the output signals. The grip assessment further includes a computing device communicatively coupled to the grip assessment device to receive the grip profile data from the grip assessment device. The computing device includes a processor and a memory in which grip profile comparison instructions are stored. Execution of the grip profile comparison instructions causes the processor to implement a comparison of the grip profile data with preset grip profile data, and provide a grip profile assessment based on the comparison.

In accordance with still another aspect of the disclosure, a ball includes a support structure having a periphery, a cover positioned around the support structure and configured to define an exterior surface of the ball, a plurality of sensor elements disposed along the periphery of the support structure, each respective sensor element of the plurality of sensor elements being configured to generate an output signal indicative of force applied to the exterior surface at the respective sensor element, a motion sensor supported by the support structure, the motion sensor being configured to measure motion of the ball influenced by the force applied to the exterior surface, and a control circuit communicatively coupled to the plurality of sensor elements and the motion sensor to generate data based on the output signals and the measured motion.

In accordance with still another aspect of the disclosure, a method of assessing dynamics of a ball includes capturing, with a plurality of sensor elements embedded in the ball, sensor data indicative of a spatial distribution of forces applied to an exterior surface of the ball, capturing, with a motion sensor embedded in the ball, motion data indicative of motion of the ball influenced by the forces applied to the exterior surface of the ball, and providing, with a control circuit embedded in the ball, the sensor data and the motion data to a processor external to the ball.

In connection with any one of the aforementioned aspects, the devices, systems, and/or methods described herein may alternatively or additionally include any combination of one or more of the following aspects or features. The plurality of sensor elements are disposed between the support structure and the cover. Each sensor element of the plurality of sensor elements includes a discrete sensor. The grip assessment device further includes a sensor film disposed between the cover and the support structure. The plurality of sensor elements are arranged as a grid of sensor elements disposed on the film. Each sensor element of the plurality of sensor elements is configured to measure pressure. Each sensor element of the plurality of sensor elements includes a piezoelectric sensing element. The grip assessment device further includes a circuit disposed inside the support structure, communicatively connected to the plurality of sensor elements, and configured to generate grip profile data based on the output signals. The circuit is configured for wireless communication of the grip profile data. The support structure includes a core. The cover includes a polymeric shell in which the core and the plurality of sensor elements are embedded, and a cover layer wrapped around the polymeric shell. The core and the polymeric shell are configured such that the grip assessment device has inertial characteristics of a baseball. The support structure is ball-shaped. The plurality of sensor elements includes a flexible, spiral-shaped film wrapped about the periphery of the support structure. The plurality of sensor elements includes a flexible film wrapped about the periphery of the support structure. The flexible film includes a plurality of petals. The support structure is handle-shaped. The support structure is spheroid-shaped and the periphery has a plurality of flattened sections, each flattened section of the plurality of flattened sections having a respective sensor element of the plurality of sensor elements disposed thereon. The grip assessment device further includes a plurality of cables. The support structure has a plurality of openings in the periphery. Each sensor element of the plurality of sensor elements includes a respective cable of the plurality of cables. Each respective cable of the plurality of cables passes through a respective opening of the plurality of openings. The instrumented baseball further includes a polymeric shell in which the core and the plurality of sensor elements are embedded. The leather cover is wrapped around the polymeric shell. The core is spheroid-shaped and the periphery has a plurality of flattened sections, each flattened section of the plurality of flattened sections having a respective sensor element of the plurality of sensor elements disposed thereon. The method further includes obtaining, with the processor, pitch data captured during the pitch, the pitch data being indicative of a pitch track taken by the instrumented baseball, implementing a further comparison of the pitch track data with preset pitch track data for a pitch type of the pitch, and providing the assessment includes providing, with the processor, information regarding the further comparison. The motion sensor includes an inertial measurement unit. The data generated by the control circuit comprises spin data indicative of a spin rate of the motion. The data generated by the control circuit includes velocity data indicative of a velocity of the motion. The data generated by the control circuit includes trajectory data indicative of a trajectory of the motion. The motion sensor includes an inertial measurement unit. The method further includes calculating, with a processor, trajectory data based on the motion data. The trajectory data includes a spin rate of the motion, a spin axis of the motion, a velocity of the motion, and a break of the motion.

### BRIEF DESCRIPTION OF THE DRAWING FIGURES

For a more complete understanding of the disclosure, reference should be made to the following detailed description and accompanying drawing figures, in which like reference numerals identify like elements in the figures.
Figure 1 is a schematic view and block diagram of a grip assessment device in accordance with one example.
Figure 2 is a schematic view and block diagram of a grip assessment system and device thereof in accordance with another example.
Figure 3 is a flow diagram of a grip assessment method in accordance with one example.
Figure 4 is a flow diagram of a method of manufacturing a grip assessment device in accordance with one example.
Figure 5 is a schematic, perspective, partial view of a core of a grip assessment device in accordance with one example.
Figure 6 is a perspective, photographic, partial view of a core of a grip assessment device in accordance with another example.
Figure 7 is a plan, photographic view of a sensor element of a grip assessment device in accordance with one example.
Figure 8 is a perspective, photographic view of a grip assessment device under construction in which a core having a plurality of sensor elements disposed thereon in accordance with one example.
Figure 9 is a perspective, photographic view of a grip assessment device under construction in which a core and a plurality of sensor elements are embedded in a polymeric shell in accordance with one example.
Figure 10 is a perspective, photographic view of a grip assessment device after a construction process ends with a wrapping of a leather cover in accordance with one example.
Figure 11 is a plot of calibration data for a sensor element in which a sensor output signal is plotted as a function of applied load.
Figure 12 is a schematic, plan view of a spiral-shaped, planar sensor circuit configured for disposition across a spherical or other three-dimensional curved periphery in accordance with one example.
Figure 13 is a schematic, perspective of the planar sensor circuit of Figure 12 after disposition across a spherical periphery.
Figure 14 depicts schematic, plan views of petal-shaped, planar sensor circuits configured for disposition across a spherical or other three-dimensional curved periphery in accordance with one example.
Figure 15 is a schematic, perspective of one of the planar sensor circuits of Figure 12 after disposition across a spherical periphery.
Figure 16 is a schematic, plan view of a planar sensor circuit distributed over a set of hexagonal and pentagonal panels (i.e., in a truncated icosahedron or soccer ball configuration) for disposition across a spherical or other three-dimensional curved periphery in accordance with one example.

The embodiments of the disclosed devices, systems, and methods may assume various forms. Specific embodiments are illustrated in the drawing and hereafter described with the understanding that the disclosure is intended to be illustrative. The disclosure is not intended to limit the invention to the specific embodiments described and illustrated herein.

### DETAILED DESCRIPTION OF THE DISCLOSURE

Devices, systems, and methods of sensing and assessing grip profiles are described. The disclosed devices include a grip assessment device having multiple sensor elements distributed across a grip surface. The disclosed devices may thus be capable of measuring grip strength around or across the entirety of the grip surface. The multiple sensor elements measure each force applied to the grip surface. The data provided by the multiple sensor elements may be compiled to provide the grip profile, which may include a total magnitude of force to the ball. The grip profile includes both grip position data and grip magnitude data.

In some cases, the grip assessment device is an instrumented baseball or other ball. The instrumented baseball is capable of measuring forces (e.g., the normal forces) applied to the surface of the ball. The plurality of sensor elements provide output signals that map the forces to discrete locations on the ball. The instrumented baseball may thus be used to determine the grip force profile applied to the ball. The disclosed devices, systems, and methods may thus address the general lack of knowledge regarding, for instance, the precise locations on a baseball where force is applied (e.g., with various grips for different pitches) and/or where forces applied to the ball originate.

In some cases, the instrumented baseball (or other ball) has the look and feel of a real baseball (or other ball). For instance, the instrumented baseball (or other ball) may be capable of being thrown, and have the surface, inertial, and other apparent characteristics of a baseball (or other ball). In these and other cases, the sensor elements and other active components of the instrumented baseball may be embedded despite the challenge of fitting the components inside the baseball. To the user, the instrumented ball may thus appear to be in all aspects (e.g., feel, weight, functionality, etc.) identical to a conventional, non-instrumented ball.

The grip force profile provided by the instrumented baseball may be used to measure and otherwise characterize the different grips used by a pitcher or pitchers for various pitches (i.e., pitch types). The grip force profile may also be used to quantify the muscular effort required by a pitcher or pitchers to successfully execute the various pitches. By measuring the force applied to the surface of the ball, the instrumented baseball may be used to quantify how different grips affect the outcome of a pitch (speed, spin, and break) for different pitchers. The instrumented baseball is thus useful as a training or coaching tool. For example, the data provided by the instrumented ball may be used to measure the different grips used by a pitcher for different pitches.

The grip profile information provided by the instrumented baseball is also useful as a tool to analyze the biomechanical characteristics of a pitcher and/or particular pitches. For example, by mapping the force on the baseball, the disclosed devices, systems, and methods may be used to determine the exertion applied by, e.g., the flexor wad. In quantifying the muscular effort involved in executing various pitches, the disclosed devices, systems, and methods may be used to determine or correlate which pitches are most fatiguing to the flexor wad (forearm musculature). Other biomechanical characteristics may be determined for various analyses, including, for instance, fatigue analysis, injury prevention, and other injury identification.

Although described herein in connection with instrumented baseballs, the nature, construction, configuration, characteristics, and other aspects of the grip assessment device may vary with the grip surface thereof. For instance, the grip assessment device may be applied to other types of balls, such as softballs and footballs. Objects other than balls may be configured with or as a grip assessment device. For instance, in other sports-related contexts, the grip assessment device may be a club (e.g., golf club), a racket (e.g., tennis racket), or a bat (e.g., baseball bat). The disclosed devices, systems, and methods are also not limited to sports-related contexts. The grip assessment device may be, include, or involve any object having a grip surface for which grip profile data is useful (e.g., an object for which the profile of a grip of the object is outcome-determinative). Examples of such non-sports related equipment or instrumentation include a steering wheel, a bicycle handle, various consumer products, handheld controllers for use in the workplace and elsewhere, and other objects, The disclosed devices, systems, and methods may accordingly be applied to any object having a grip surface.

Regardless of the nature of the object being gripped, the disclosed devices may be configured such that the operational characteristics of the object are not adversely or otherwise affected by the integration of the components of the disclosed devices. For instance, the ball or other object may thus appear to be identical to an actual, non-instrumented instance of the ball or other object. To these ends, a circuit of the ball or other object may use battery power and/or wireless communications to acquire and transmit grip position data, grip magnitude data, and other data without affecting the operational characteristics of the ball or other object.

Although described in connection with injury prevention, the disclosed devices, systems, and methods are also useful in connection with sports and other performance training. For instance, knowing the position of the fingers and palm, and the respective pressure distribution, is a useful tool for refining sport-specific throwing technique. In some cases, the disclosed devices, systems, and methods are capable of supporting changes in technique on-field via, e.g., immediate feedback.

The disclosed devices, systems, and methods are not limited to applications involving measurement of grip forces on a ball. For instance, the disclosed devices, systems, and methods may be directed to assessing the dynamics of any ball to which surface forces are applied. Data indicative of the surface forces is captured, along with motion data indicative of motion of the ball influenced by the surface forces. The nature of the surface forces may vary. In some cases, the surface forces are applied by a human appendage (e.g., a foot striking a soccer ball, or a hand striking a volleyball) or an instrument (e.g., a golf club striking a golf ball, or a baseball bat striking a baseball). The characteristics of the ball may vary accordingly.

The disclosed devices, systems, and methods are thus useful in connection with multiple types of objects. In some cases, the disclosed devices may be configured as any type of object having a grip surface. In such cases, the disclosed devices may or may not be a ball, and motion data indicative of motion influenced by the grip forces may or may not be captured. In cases in which the disclosed devices are configured as a ball, the surface forces may or may not be grip forces, and motion data may or may not captured. The corresponding systems and methods may vary accordingly. Therefore, references herein to grip assessment, grip forces, and other grip-related aspects in connection with the disclosed devices, systems, and methods should be understood to include or involve other surface forces when in the context of balls having the embedded sensor functionality described herein.

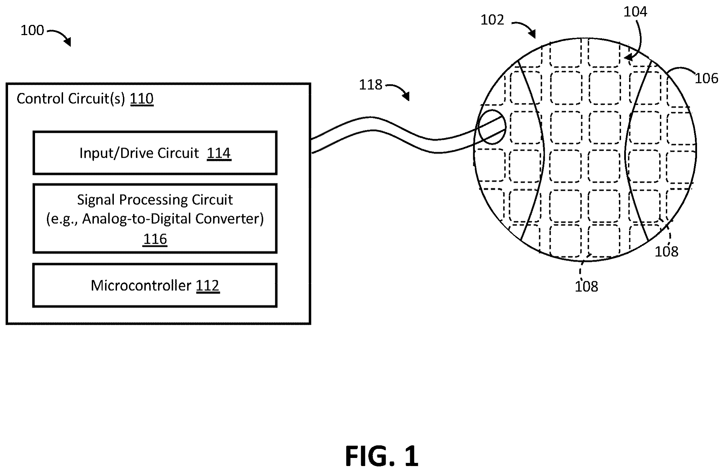

Figure 1 depicts a grip assessment device 100 in accordance with one example. In this case, the grip assessment device 100 is or includes a baseball 102 that can measure the magnitude and location of an applied grip. In other cases, the grip assessment device 100 is or includes another type of ball, such as a football, or other gripped object. In still other cases, the device 100 may be any type of ball (with or without a grip surface) with embedded sensor functionality for capturing surface and motion data, as described herein. The device 100 is depicted schematically in Figure 1 for ease in description. Non-schematic depictions of example grip assessment devices are shown and described in connection with other drawing figures.

The device 100 includes a core 104 and a cover 106 positioned around the core 104. The core 104 has a periphery. The periphery may be shaped or otherwise configured to support the grip assessment functionality, as described below. In some cases, the periphery and/or other aspects of the core 104 may be customized via three-dimensionally printing or other rapid-prototyping. The cover 106 is configured to define a grip or other exterior surface, e.g., outside of the periphery of the core 104. In this case, the core 104 is ball-shaped, and the cover 106 is or includes a leather cover.

The shape, configuration, composition, and other characteristics of the core 104 and the cover 106 may vary from the baseball 102 of Figure 1. For example, the core 104 may be cylindrically shaped. The core 104 may be or include any type of inner structure, support, or support structure. For instance, the core 104 may be, or otherwise include, a bladder of, e.g., a football, basketball, or other inflated ball or object.

Examples of the core 104, the cover 106, and aspects thereof are further shown and described in connection with other drawing figures. The cover 106 may include a number of layers, including, for instance, an inner shell and an outer layer.

The device 100 further includes multiple sensor elements 108 disposed along the periphery of the core 104. The sensor elements 108 are supported by the core or support structure 104. Each respective sensor element 108 is configured to generate an output signal indicative of force applied to the grip surface at the respective sensor element 108. The sensor elements 108 are distributed across the grip surface such that the output signals from the sensor elements 108 are collectively indicative of a grip profile along the grip surface. The grip profile providing grip position data and grip magnitude data correlated with the grip position data, as described below. The device 100 may include one or more additional sensors, including, for instance, a motion sensor, such as an inertial measurement unit, for capturing motion data as described herein.

In the example of Figure 1, each sensor element 108 is a discrete sensor. Each sensor element 108 is configured to generate its output signal independently of the other sensor elements 108. In other cases, the sensor elements 108 may be integrated in, for example, a grid or other framework. The extent or nature of the integration may vary.

The number and layout of the sensor elements 108 may vary. In one baseball example, the number of sensor elements 108 falls in a range from about 30 to about 40 (e.g., 32 sensor elements), but the number may vary with other sensor layouts. The layout may differ from the rectilinear arrangement shown in Figure 1. For instance, the sensor elements 108 may be oriented in a diamond arrangement or other arrangement well-suited for the shape of each sensor element 108.

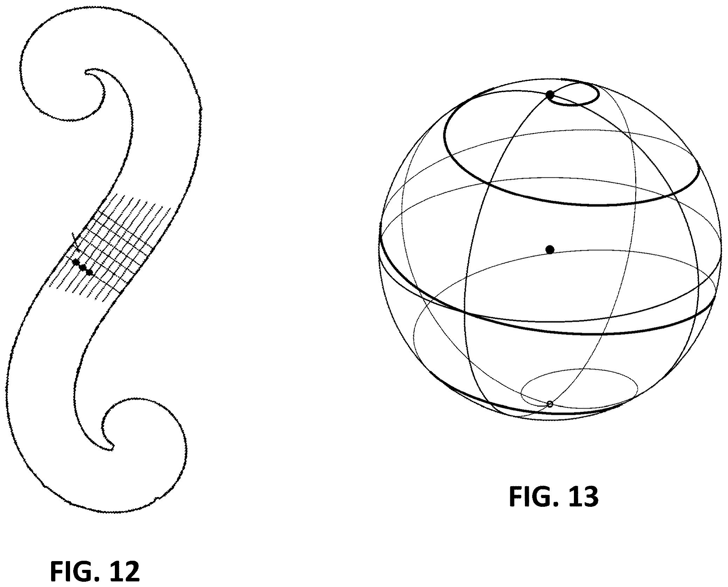

The size, number, shape and layout of the sensor elements 108 may be selected to maximize or increase the extent to which the grip surface is covered by the sensor elements 108. Increasing the coverage area provides a more accurate and useful grip profile. Completely covering a spherical or spheroid surface is challenging. To that end, placing many small sensors around the surface of the baseball 102 may be useful. The sensor elements 108 may be carried on a flexible circuit or other film shaped to allow for universal or otherwise substantial coverage of the surface. Examples of such films are described below in connection with Figures 12-16.

Each sensor element 108 may be or include one or more force-sensitive resistors. In some cases, each sensor element 108 is or includes a piezoelectric sensing element. Each sensor element 108 may be configured to measure pressure. For instance, each sensor element 108 may measure pressure via compression or other physical deformation of one or more layers of the cover 106. Other types of pressure or force sensors may be used, including, for instance, capacitive sensors.

The sensor elements 108 may be embedded in a cover layer or other component of the baseball 102. For example, the sensor elements 108 may be encased in a flexible polymer shell (or other cover layer) after being secured to, mounted on, or otherwise disposed on the core 104. In some cases, the polymer shell or layer is or includes polyurethane, which may be applied via a mold. The polymer layer may be considered to be one of multiple layers of the cover 106. The leather or other cover layer of the cover 106 may then be wrapped around the polymer layer.

The grip assessment device 100 includes a control circuit 110 communicatively coupled to the sensor elements 108 and configured to generate grip profile data based on the output signals. The control circuit 110 may receive the sensor elements 108 directly or indirectly from the sensor elements 108. In the example of Figure 1, the control circuit 110 is disposed outside of the baseball 102 for ease in implementation and/or depiction. In other cases, one or more components of the control circuit 110 are embedded or otherwise disposed inside the core 104 or the baseball 102. The control circuit 110 may be coupled to the sensor elements 108 via one or more wired connections.

In the example of Figure 1, the control circuit 110 includes a microcontroller 112, an input circuit 114, and a signal processing circuit 116. These components of the control circuit 110 may be integrated to any desired extent. For instance, the components may be provided via a system-on-a-chip (SoC) or other integrated architecture. Any number of integrated circuit (IC) or other components may be used to realize the components of the control circuit 110. Additional, fewer, or alternative components may be provided. For instance, the control circuit 110 may include a separate circuit directed to providing digital output signals, i.e., the grip profile data, from the microcontroller 112. The control circuit 110 may include additional, fewer, or alternative components. For instance, the control circuit 110 may include a battery and/or other power source. The battery may be embedded or otherwise internally disposed.

The output signals are received via the input circuit 114. The input circuit 114 may be configured to develop an analog representation of the force applied to each sensor element 108. For example, the input circuit 114 may include a resistor or resistive element across which a voltage is developed in accordance with the current passing through the sensor element 108. In one example, each output signal is measured across a 150 Ω resistor. A respective resistor or resistive element may be provided for each sensor element 108. Alternatively, the input circuit 114 may be or include a multiplexer or other arrangement. In some cases, the input circuit 114 may also be configured to drive (e.g., bias), or support the driving of, the sensor elements 108.

The signal processing circuit 116 may be configured to process the analog signals provided via the input circuit 114. In some cases, the signal processing circuit 116 is or includes an analog-to-digital converter. Alternative or additional processing may be provided. For instance, the signal processing circuit 116 may be directed to filtering or other conditioning of the analog or other signals provided by the input circuit 114 or the sensor elements 108.

The microcontroller 112 receives the digital or other signals from the other components of the control circuit 110. The microcontroller 112 may be configured to generate or record data indicative of the magnitude of the force applied to each sensor element 108, along with data indicative of the location thereof, i.e., the location of the corresponding force sensor 108. The force magnitude and location data generated by the microcontroller 112 may be used to understand different grips and to quantify different grip profiles.

The microcontroller 112 may be or include a processor, and may be realized on an IC chip. The processor may be programmed via instructions stored in one or more memory units. The one or more memory units may or may not be integrated with the processor, e.g., onboard the IC chip. In one example, the microcontroller 112 is or includes an Arduino Uno processor. Other microcontrollers or processors may be used. In some cases, the control circuit 110 may include one or more multiplexers to effectively increase the number of analog input ports (e.g., pins) provided by the microcontroller 112.

The Arduino Uno or other microcontroller 112 may be configured to sort and display the grip profile data. For instance, the microcontroller 112 may include or be coupled to a serial monitor on which the grip profile data is displayed. The microcontroller 112 may be configured to implement alternative or additional procedures. For example, drift present during read time may be accommodated or compensated for or otherwise addressed by a procedure executed by the microcontroller 112, e.g., via instructions stored in the one or more memory units).

The example of Figure 1 depicts a wired connection 118 between the sensor elements 108 and the control circuit 110. The grip profile data may be obtained from the control circuit 110 via an additional wired connection. For instance, the microcontroller 112 and/or, more generally, the control circuit 110, may have one or more output ports to which a cable(s) can be connected.

In other cases, the grip profile data may be obtained from, and provided by, the control circuit 110 wirelessly. In such cases, the control circuit 110 may be embedded or otherwise disposed inside the baseball 102. For instance, the control circuit 110 may be disposed within an interior space of the core 104. The grip assessment device 100 may thus a fully wireless instrumented baseball capable of being thrown. As a result, the grip assessment device 100 may also be configured to have identical inertial properties to a regulation baseball. In this way, the grip assessment device 100 provides the ability to understand the relationship between different grips and pitch performance.

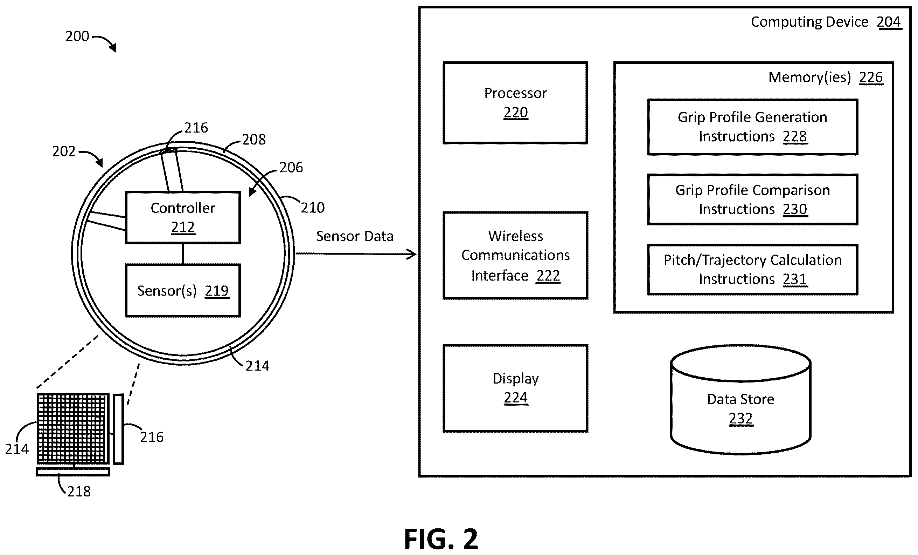

Figure 2 depicts a grip assessment system 200 in accordance with one example. The grip assessment system 200 includes a grip assessment device 202 and a computing device 204 communicatively coupled with the grip assessment device 202. The grip assessment device 202 may have one or more features in common with the other devices described herein, including, for instance, the device 100 of Figure 1. The computing device 204 may be used in conjunction any of the devices described herein.

In some cases, the grip assessment device 202 is or includes any type of ball, such as a baseball, football, soccer ball, volleyball, or golf ball. The ball may or may not have a gripped surface. In cases not involving a grip surface, the system 200 may be directed to assessing a spatial distribution of forces applied to an exterior surface of the ball, as in, e.g., a ball dynamics assessment system. In still other cases, the device 202 may be any type of gripped object, such as a racket, steering wheel, or handheld controller.

The grip assessment device 202 may have a spherical, spheroid, cylindrical, or other shaped grip or other exterior surface. The curvature of the grip surface may vary accordingly. The grip assessment device 202 may have one or more features or aspects in common with other grip assessment devices described herein. For instance, the grip assessment device 202 may include a core 206 (or support structure) having a periphery, a cover 208 positioned around the core 206 and configured to define a grip surface 210, and multiple sensor elements disposed along the periphery of the core 206 and disposed between the core 206 and the cover 208. In this example, the cover 208 includes a single layer, e.g., a leather cover, but any number or type of cover layers may be used, including, for instance, polymeric inner shell, but alternative or additional cover layers may be included.

The grip assessment device 202 includes a controller 212 disposed in the core 206, e.g., within an interior space of the core 206. The embedded nature of the controller 212 allows the grip assessment device 202 to be gripped and used in a natural manner. The controller 212 may be configured in a manner similar to the control circuit described hereinabove. For instance, the controller 212 may be or include circuitry configured to generate grip profile data based on the output signals from the sensor elements. In this case, the controller 212 uses a wireless communication link via, e.g., Bluetooth or another wireless communication standard, with the computing device 204 to transfer the sensor data (e.g., grip profile data) from the grip assessment device 202 to the computing device 204.

The grip assessment device 202 includes multiple sensor elements distributed across the grip surface 210, as in the above-described examples. The example of Figure 2 differs from the above-described examples in that the sensor elements are disposed in a sensor grid 214. The sensor elements of the sensor grid 214 are thus not discrete sensors. Instead, the sensor elements are arranged and interconnected in rows and columns of the sensor grid 214. The sensor grid 214 includes row and column read/drive circuitry 216, 218 coupled to the sensor elements and configured to drive and/or read the output signals of the sensor elements.

The sensor grid 214 may be configured as a flexible sheet or film, which may be wrapped around the periphery of the core 206. Each sensor element may be formed or otherwise disposed on the film. For example, the film may act as a substrate for transistor, resistive, capacitive, and/or other circuit elements of the sensor grid 214.

The sensor grid 214 may be well-suited for examples in which the grip assessment device 202 is cylindrical or otherwise handle-shaped, such as a golf club. In other cases, the film may be stretchable and/or shaped to accommodate other device shapes (e.g., ball-shaped or other spheroid devices).

In the example of Figure 2, the grip assessment device 202 includes one or more motion sensors 219 configured to capture or measure motion of the grip assessment device 202. The motion is influenced by the grip or other forces applied to the exterior surface of the device 202. In cases in which the grip assessment device 202 is or includes a ball, the sensor(s) 219 may be configured to capture data indicative of a trajectory (e.g., pitch track) of the motion. For example, the motion sensor(s) 219 may be or otherwise include an inertial measurement unit (IMU). The motion sensor(s) 219 may include an accelerometer, a gyroscope, and/or other motion sensors. The extent to which the motion sensors 219 are integrated may vary as desired.

The computing device 204 is communicatively coupled to the grip assessment device 202 to receive the grip profile data from the grip assessment device 202. The computing device 204 may be a portable computing device, such as a smart phone, or a personal computing device, such as a laptop or desktop. The computing device 204 includes a processor 220 process the grip profile data, a wireless communications interface 222 to receive the grip profile data, and a display 224 to provide the raw or processed grip profile data to a user. The computing device 204 further includes a memory 226 in which instructions for the processor 220 are stored. In this example, grip profile generation instructions 228, grip profile comparison instructions 230, and motion (e.g., pitch track or other trajectory) calculation instructions 231 are stored in the memory 226. The instructions 228, 230, 231 may be integrated to any desired extent. Additional, fewer, or alternative instructions may be stored. For instance, the generation of the grip profile (or grip profile data) may be entirely handled by the controller 212. The memory 226 may include one or more memories or memory units.

Execution of the grip profile comparison instructions 230 causes the processor 220 to implement a comparison of the grip profile data with preset grip profile data and provide a grip profile assessment based on the comparison. The preset grip profile data may be stored in a data store 232 of the computing device 204. In some cases, the data store 232 may be located on a server or other remote computing device.

The grip profile assessment may be directed to assessing to what extent the grip profile is appropriate for a given pitch or other activity, e.g., as a training tool. The grip profile assessment may be alternatively or additionally be directed to injury prevention, injury detection, rehabilitation, and/or other uses.

Execution of the motion calculation instructions 231 causes the processor 220 to generate data based on the motion data measured or captured by the motion sensor(s) 219. For example, the data may include spin data indicative of a spin rate of the motion and/or a spin axis of the motion. Alternatively or additionally, the data may include velocity data indicative of a velocity of the motion, and/or trajectory data indicative of a break or other trajectory of the motion. The data calculated via execution of the motion calculation instructions 231 may allow a pitch or other object trajectory to be analyzed in conjunction with the spatial distribution of the grip or other surface forces without reliance on further equipment, such as a video camera and the associated video analysis system.

The computing device 204 may be used in conjunction with any of the other grip assessment devices described herein.

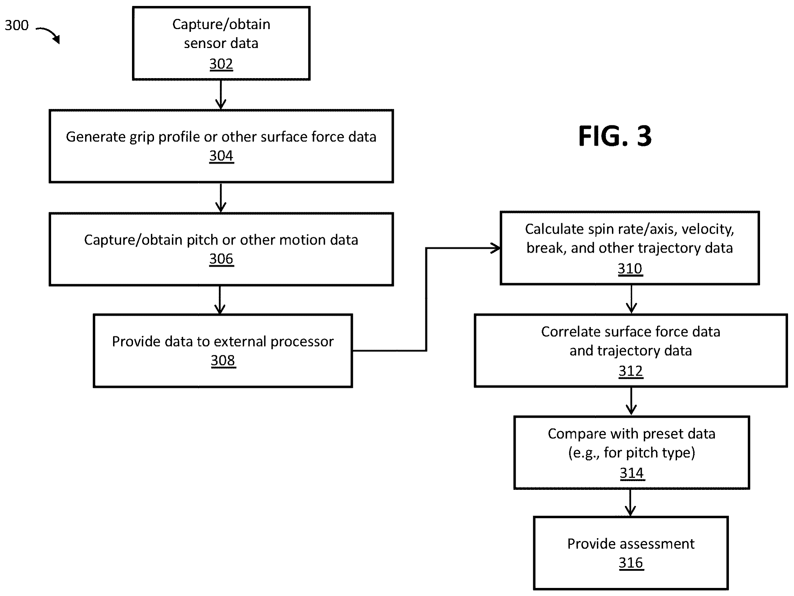

Figure 3 depicts a method 300 of assessing a grip profile, e.g., a baseball grip profile, or other spatial distribution of surface forces, in accordance with one example. One or more acts of the method 300 may be implemented by any one or more of the controllers, control circuits, or processors described herein, and/or another controller, control circuit, or processor. For example, some or all of the method 300 may be implemented as a result of the execution of the grip profile comparison instructions 230 (Figure 2) by the processor 220 (Figure 2). Alternatively or additionally, one or more acts of the method 300 may be implemented by a microcontroller or other controller of one of the grip assessment devices described herein. For example, grip profile data may be generated by the grip assessment device.

The method 300 may also be directed to assessing the dynamics of the motion of a ball. For example, the dynamics of the ball motion may be calculated via the execution of the motion calculation instructions 231 (Figure 2) by the processor 220 (Figure 2).

The method 300 may begin with one or more acts directed to capturing sensor data indicative of the grip profile or other spatial distribution of forces applied to an exterior surface. The sensor data is captured by a plurality of sensor elements embedded in the ball or other object as described herein. Any one of the disclosed grip assessment devices may be used to capture the sensor data. In the example of Figure 3, the method 300 includes an act 302 in which sensor data is obtained. For example, the sensor data may be obtained from the grip assessment device via a wired or wireless communication. In baseball examples, the sensor data may be captured during a pitch of an instrumented baseball. The sensor data may be indicative of grip position data and grip magnitude data correlated with the grip position data, or other spatial distribution of forces applied to the exterior surface.

In an act 304, grip profile data (or other surface force profile data) is generated based on the sensor data. The grip profile data may be generated via filtering, conditioning, or other data processing of the sensor data.

In the example of Figure 3, pitch or other motion data is also captured and/or obtained in an act 306. The motion data is captured with one or more motion sensors (e.g., an inertial measurement unit) embedded or otherwise disposed in the ball or other object. The motion is influenced by the forces applied to the exterior surface. The pitch data may be indicative of a track, i.e., a pitch track or trajectory, taken by the grip assessment device while the sensor data is captured for the grip profile. Alternatively or additionally, the pitch data is indicative of a speed of a pitch executed while the sensor data is captured for the grip profile. Additional, fewer, or alternative characteristics of the pitch may be provided via the pitch data. The pitch data may be captured by a separate device, e.g., a radar gun or other pitch tracking device. The pitch data may be captured with or without involving the grip assessment device.

The grip profile data and the motion data may then be provided to an external processor in an act 308. The external processor is external to the ball or other object to which the surface forces are applied. The data may be provided wirelessly. The manner in which the data is transmitted may vary. The act 308 may include the compilation, association, correlation of other processing of the data before the transmission. In some cases, the remainder of the method 300 may accordingly be implemented by the external processor. The acts up to and including the act 308 may be implemented by a control circuit, controller, or other processor embedded in the ball or other object to which the surface forces are applied.

The motion data may be processed in an act 310 to calculate one or more characteristics of the motion. For example, pitch or other trajectory data may be calculated.

The nature of the trajectory data may vary. The trajectory data may include, for instance, a spin rate of the motion, a spin axis of the motion, a velocity of the motion, and a break of the motion.

In the example of Figure 3, the trajectory data is correlated and/or otherwise processed with the grip profile or other surface force data in an act 312. For example, the processing of the trajectory data and the surface force data may include or involve the calculation of one or more parameters.

In an act 314, a comparison of the calculated parameters, trajectory data, and/or surface force data (e.g., grip profile data) with preset data is implemented. The comparison may include any number of comparisons or other analyses of the calculated and/or measured data with corresponding preexisting data. In baseball examples, the preset pitch track data and, thus, the comparison may be specific to a particular pitch type. For instance, the pitch type may determine the preset data to be used in the comparison.

An assessment of the grip profile or other surface force distribution is then provided in an act 316 based on the comparison. The assessment may include information regarding the comparison involving the motion data, the surface force data, and/or the parameters or characteristics calculated therefrom. The assessment may be provided via a display, such as the display 224 (Figure 2) of the computing device 204 (Figure 2).

The method 300 may include additional, fewer, or alternative acts. For instance, the method 300 may not include acts relating to pitch data.

The acts of the method 300 may be implemented in an order differing from the order shown in Figure 3. For instance, the act 306 may be implemented simultaneously with, or before, the act 302.

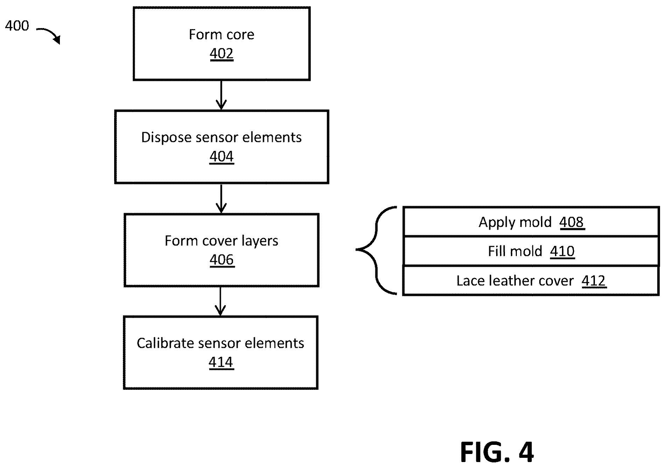

Figure 4 depicts a method 400 of manufacturing a grip assessment device in accordance with one example. The method 400 may be used to manufacture any of the grip assessment devices described herein or another grip assessment device. The method 400 may include additional, fewer, or alternative acts. For instance, the method 400 may include one or more acts directed to disposing or integrating a controller of the grip assessment device, e.g., within an interior thereof.

The method 400 may begin in an act 402 in which a core is formed. In some cases, the core is formed via three-dimensional printing. Alternative or additional processes may be used. The core may be formed from multiple parts (e.g., two halves) that are snapped together or otherwise joined. The two halves may be joined after implementation of one or more acts of the method 400.

In an act 404, multiple sensor elements are affixed to, or otherwise mounted on, the core. In some cases, each sensor element may be individually attached or applied to the core. Application of each sensor element may include affixing a sensing or sensor portion on the core and feeding of a flexible cable or other connectors through a slot of other hole in the core. The sensor area may be attached to the core with an epoxy or other adhesive material. In other cases, the sensor elements are applied collectively as, e.g., a sheet or film.

One or more cover layers are then formed in an act 406. The cover layers may include a polymeric inner shell or layer and a leather cover, as described above. The polymeric shell or layer may be formed via a casting process in which a mold (e.g., a two-part mold) is applied in an act 408, and filled with, e.g., polyurethane, in an act 410. In one example, the casting material used is VytaFlex 50 polyurethane, but other polymeric materials may be used. The mold may be used to enclose or embed the sensor elements and achieve a desired shape (e.g., spherical). The leather cover may then be wrapped and laced over the polymeric layer in an act 412.

The leather cover may be positioned such that the laces are aligned with, or otherwise disposed in accordance with, the positions of the sensing elements.

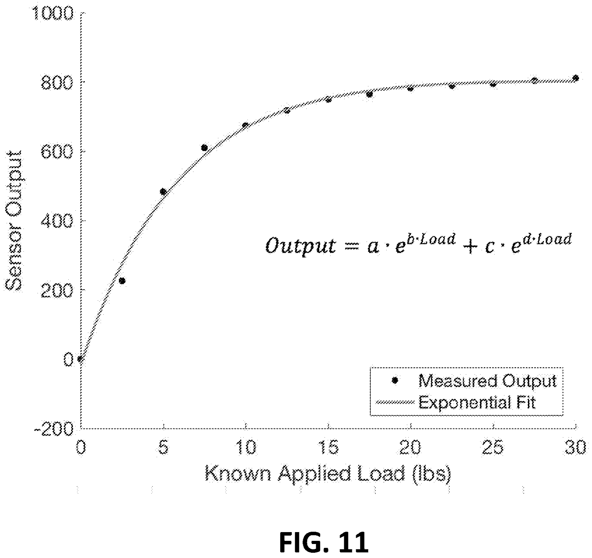

Calibration of the sensor elements may then be implemented in an act 414. Further details regarding an example calibration procedure are provided below in connection with Figure 11.

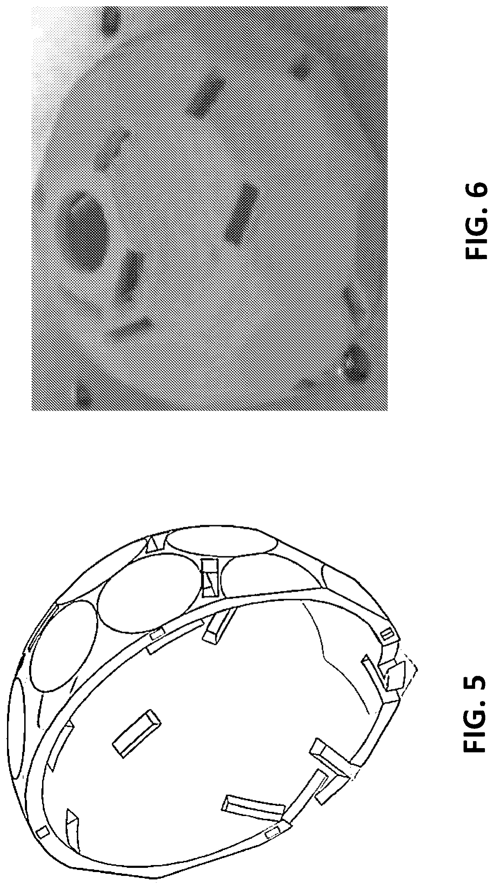

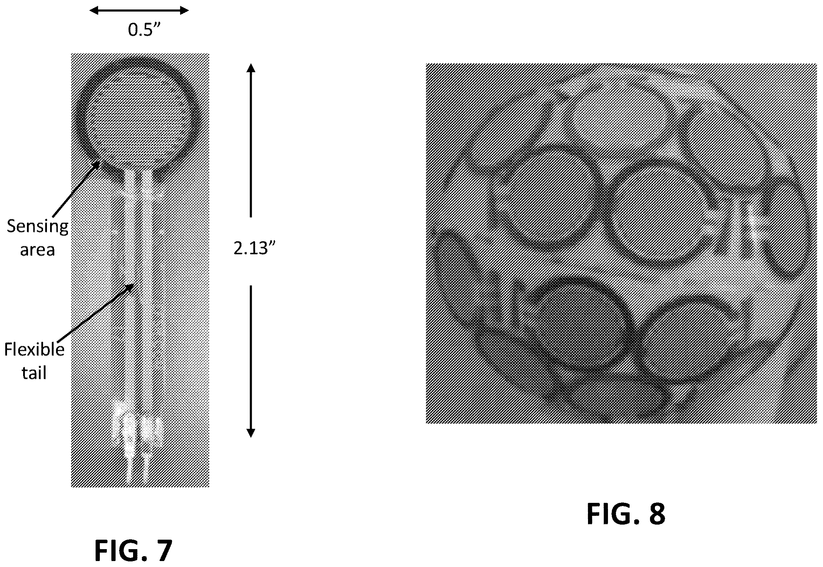

Figures 5 and 6 depict examples of a core portion of an instrumented baseball. In each case, the core is spheroid-shaped. The periphery of the core has a plurality of flattened sections. Each flattened section is configured for a respective sensor element of the plurality of sensor elements to be disposed thereon. The periphery of the core also has multiple slots or holes for a flexible cable or other wiring for each sensor element. In these cases, each slot is adjacent to a respective one of the flattened sections. Each respective cable passes through a respective opening of the plurality of openings, as shown in Figure 8.

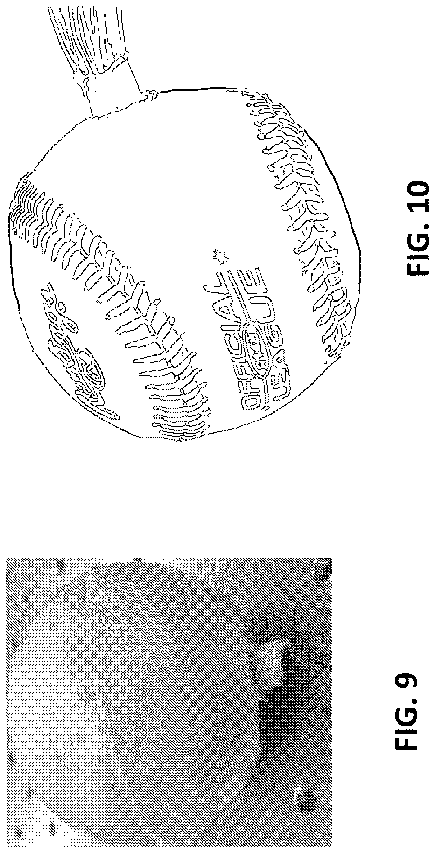

In the example of Figure 6, the core includes a circular hole for wiring to connect to an external control circuit. Figures 9 and 10 depict examples having wiring exiting a hole in both the core and the cover of an instrumented baseball. The circular hole may not be included in cases in which an internal or integrated control circuit is used.

Figure 7 depicts an example of a sensor element for placement on a core of a grip assessment device. In this example, the sensor is or includes a thin piezoelectric pressure sensor. The piezoelectric sensor is disposed in a sensing area. The sensor element further includes a cable extending from the sensor area as a tail. The cable may include a flexible substrate on which conductive traces are carried. In this case, each trace terminates in a respective lead. The configuration, construction, composition, and other characteristics of the sensor elements may vary considerably from the example shown.

Figures 8-10 depict an example of an instrumented baseball at various stages of fabrication. In Figure 8, the sensor elements have been affixed to the core, and the cables of the sensor elements have been fed through the slots in the core. In Figure 9, a polymeric shell has been applied to the core. The core and the sensor elements are embedded in the polymeric shell. Figure 10 shows the embedded core after a leather cover has been wrapped around the periphery of the core and the shell to define a grip surface.

Figure 11 depicts an example of a plot of calibration data for configuring a controller or control circuit of the disclosed grip assessment devices and systems. In this example, the calibration data includes the output of the force sensitive resistors plotted as a function of the applied force. The calibration data may be obtained via a process in which known weights are applied to the sensor elements. The plot also shows a curve fitted to the calibration data. The curve may be used to generate a grip magnitude based on a given sensor output level. In this example, a second order exponential curve is used, but other curves may be used in other cases. The curve may be used to map a respective sensor output level to a value in, for instance, pounds. Alternatively or additionally, a lookup table based on the curve may be used to generated the force data.

The calibration data may be obtained via a test structure configured to apply a known weight in a concentrated manner, so as to simulate a finger. The test structure is configured to apply a range of loads to each sensor element individually.

Figures 12-16 depict examples of flexible films on which a grid or other array of sensor elements may be disposed. The flexible films may be wrapped around or otherwise disposed about the periphery of the support structure of any of the balls or other objects or devices described herein. The flexible films may be or include any type of flexible substrate upon which the sensor elements may be disposed. For example, the flexible films may be composed of, or otherwise include, a polymer material, such as thermoplastic polyurethane (TPU), but other materials may be used.

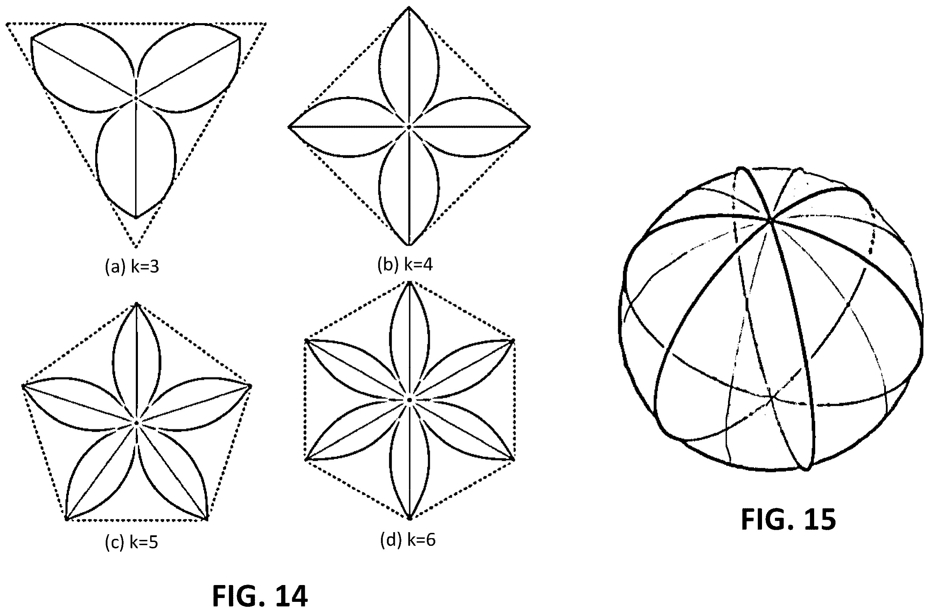

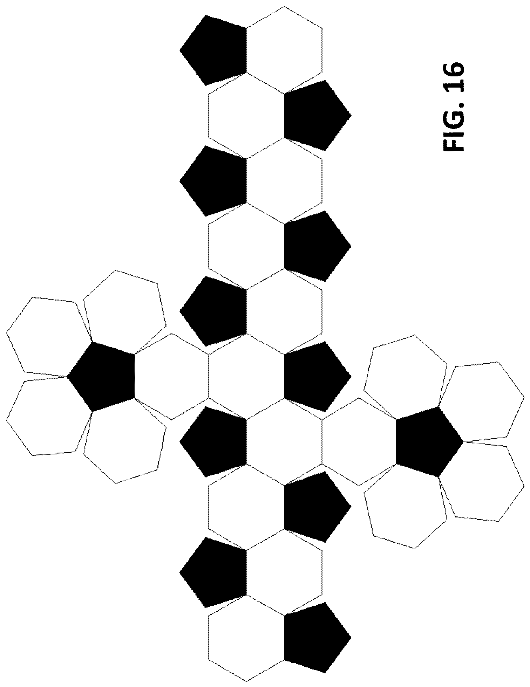

In each of the examples of Figures 12-16, the flexible film is shaped to achieve universal or substantial coverage of a spherical periphery. The flexible film may be spiral-shaped, such as a Euler spiral as shown in Figures 12 and 13. Figure 12 also schematically shows wire traces of a portion of the sensor grid. Alternatively, petal-shaped films may be used. The number of petals may vary as shown in Figure 14. Figure 15 depicts the application of an example film with six petals. In still other cases, the flexible film includes a set of hexagonal and pentagonal panels, as in, e.g., a truncated icosahedron or soccer ball configuration, as shown in Figure 16.

The present disclosure has been described with reference to specific examples that are intended to be illustrative only and not to be limiting of the disclosure. Changes, additions and/or deletions may be made to the examples without departing from the spirit and scope of the disclosure.

The foregoing description is given for clearness of understanding only, and no unnecessary limitations should be understood therefrom.

## Claims

1. A force assessment device (100)(202) comprising:
a support structure (104)(206) having a periphery, the periphery being spherical;
a cover (106)(208) positioned around the support structure and configured to define a surface (210), the cover comprising laces;
a flexible film with a plurality of sensor elements (108)(214) disposed on the flexible film, each respective sensor element of the plurality of sensor elements (108)(214) being configured to generate an output signal indicative of force applied to the surface (210) at the respective sensor element; and
a circuit (110)(212) disposed inside the support structure, the circuit (110)(212) being communicatively connected to the plurality of sensor elements (108)(214) and configured to generate force profile data based on the output signals;
wherein the plurality of sensor elements (108)(214) are distributed across the surface (210) such that the output signals from the plurality of sensor elements are collectively indicative of a force profile along the surface (210), the force profile providing force position data and force magnitude data correlated with the force position data;
**characterised in that**:
the flexible film is: i) wrapped about the periphery of the support structure (104)(206) between the cover (106)(208) and the support structure (104)(206); and ii) comprises a plurality of petals or a set of hexagonal or pentagonal panels such that the flexible film is shaped to achieve universal coverage of the periphery of the support structure (104)(206); and
the cover (106)(208) is positioned such that the laces are disposed in accordance with positions of the plurality of sensor elements (108)(214).

2. The force assessment device (100)(202) of claim 1, wherein the laces are aligned with the plurality of sensor elements (108)(214).

3. The force assessment device (100)(202) of claim 1, wherein:
the plurality of sensor elements (108)(214) are disposed between the support structure (104)(206) and the cover (106)(208); and/or
the plurality of sensor elements (108)(214) are arranged as a grid of sensor elements disposed on the flexible film.

4. The force assessment device (100)(202) of claim 1, wherein each sensor element of the plurality of sensor elements (108)(214): comprises a discrete sensor; is configured to measure pressure; and/or comprises a piezoelectric sensing element.

5. The force assessment device (100)(202) of claim 1, wherein the circuit (110)(212) is configured for wireless communication of the force profile data.

6. A method of assessing a ball force profile, the method comprising:
obtaining, with a processor, sensor data captured during an application of force to an instrumented ball, the instrumented ball comprising the force assessment device (100)(202) of claim 1, the sensor data being indicative of force position data and force magnitude data correlated with the force position data;
generating, with the processor, force profile data based on the sensor data;
implementing, with the processor, a comparison of the force profile data with preset force profile data; and
providing, with the processor, an assessment of the ball force profile based on the comparison.

7. The method of claim 6, wherein the force applied to the instrumented ball is applied by a human appendage.

8. The method of claim 6 or claim 7, wherein the instrumented ball is a soccer ball.
